(19) Europäisches Patentamt
European Patent Office
Office européen des brevets

(11) EP 2 724 735 A1

(12) EUROPEAN PATENT APPLICATION
published in accordance with Art. 153(4) EPC

(43) Date of publication:
30.04.2014 Bulletin 2014/18

(21) Application number: 12802975.8

(22) Date of filing: 22.06.2012

(51) Int Cl.:
*A61L 33/00* (2006.01) *C08F 216/06* (2006.01)
*C08F 218/08* (2006.01) *C08G 65/333* (2006.01)
*C08G 65/334* (2006.01)

(86) International application number:
PCT/JP2012/065946

(87) International publication number:
WO 2012/176861 (27.12.2012 Gazette 2012/52)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 23.06.2011 JP 2011139267

(71) Applicant: Toray Industries, Inc.
Tokyo, 103-8666 (JP)

(72) Inventors:
- SAKAGUCHI, Hirokazu
Otsu-shi, Shiga 5208558 (JP)
- SAKAGUCHI, Yuka
Otsu-shi, Shiga 5208558 (JP)
- TANAHASHI, Kazuhiro
Otsu-shi, Shiga 5208558 (JP)

(74) Representative: Kador & Partner
Corneliusstrasse 15
80469 München (DE)

# (54) MEDICAL SUPPLY

(57) An object of the present invention is to provide a medical material having a compound firmly immobilized on the surface thereof, which compound is capable of inhibiting both of the blood coagulation reactions in a primary hemostasis stage in which platelets are involved and in a coagulation thrombus formation stage in which blood coagulation factors are involved in a state retaining the anticoagulant activity. The present invention provides a medical material having a hydrophilic polymer compound immobilized on the surface thereof, in which a compound represented by the following general formula (I) and a copolymer of monomers selected from the group consisting of ethylene glycol, vinyl acetate, vinyl pyrrolidone, propylene glycol, vinyl alcohol and siloxane are bound.

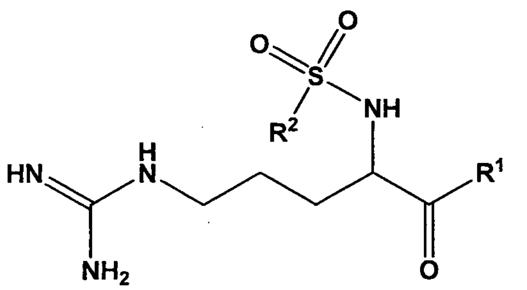

··· (I)

EP 2 724 735 A1

**Description**

TECHNICAL FIELD

The present invention relates to a medical material.

BACKGROUND ART

A blood coagulation reaction required for coagulating blood is an extremely complicated reaction in which various blood coagulation factors are involved. It is thought that a primary hemostasis stage in which platelets are involved and a coagulation thrombus formation stage in which blood coagulation factors such as thrombin are involved to stabilize and strengthen fibrins are particularly important.

The blood coagulation reaction is indispensable to lead bleeding due to injury or the like to the hemostasis. On the other hand, however, in cases where the blood coagulation reaction proceeds due to contact between the blood and a medical material, in hemodialysis or a procedure using a medical material such as catheter, stent or synthetic blood vessel, there is a risk that the formed blood clots or coagulation thrombus cause increased circulation pressure, impeded blood flow, or vascular occlusion.

As a method of decreasing these risks, known is a method of preventing blood coagulation comprising administering in advance heparin which is an anticoagulant to a patient who is to undergo hemodialysis. Yet, there are a number of problems in that excessive administration of heparin causes side effects, the control of administration dose is complicated, the method cannot be applied to a patient with hemorrhagic tendency, or the like. Further, also in a procedure using a medical material such as catheter, thrombolytic agents such as heparin or urokinase are used as necessary. Yet, the use thereof may in some cases increases the hemorrhagic tendency of a patient.

Recently, in order to avoid these problems, attempts to prevent blood coagulation during treatment by immobilizing a compound having an anticoagulant activity including heparin on the surface of medical materials such as blood circuits or the like have been reported (Patent Documents 1 to 9).

PRIOR ART REFERENCES

PATENT DOCUMENTS

Patent Document 1: Japanese Translated PCT Patent Application Laid-open No. 2003-507082
Patent Document 2: Japanese Patent Application Laid-Open Publication No. 2001-213984
Patent Document 3: Japanese Translated PCT Patent Application Laid-open No. 2004-525888
Patent Document 4: Japanese Patent Application Laid-Open Publication No. 2006-291193
Patent Document 5: WO 08/032758
Patent Document 6: Japanese Patent Application Laid-Open Publication No. 2009-225824
Patent Document 7: Japanese Patent Application Laid-Open Publication No. 2010-082067
Patent Document 8: Japanese Patent Application Laid-Open Publication No. 2007-181691
Patent Document 9: Japanese Patent Application Laid-Open Publication No. 2007-181692

SUMMARY OF THE INVENTION

PROBLEMS TO BE SOLVED BY THE INVENTION

However, as it stands now, a medical material having a compound immobilized on its surface, which compound is capable of inhibiting both blood coagulation reactions in the primary hemostasis stage in which platelets are involved and in the coagulation thrombus formation stage in which blood coagulation factors are involved has not been developed yet. Further, in conventional medical materials having a conventional compound immobilized on its surface, which compound has an anticoagulant activity, the compounds are immobilized in a state retaining sufficient anticoagulant activity and there has been a problem in that the immobilized compound dissociates from the medical material during treatment and dissolves out into the blood. Furthermore, in cases where a plurality of compounds are used for inhibiting both of the blood coagulation reactions in the primary hemostasis stage in which platelets are involved and in the coagulation thrombus formation stage in which blood coagulation factors are involved, it is necessary to control competitive adsorption between the compounds and to control the immobilization ratio and the operation for obtaining the medical material having those compounds immobilized on the surface thereof has been very cumbersome.

In view of this, an object of the present invention is to provide a medical material having a compound firmly immobilized on the surface thereof, which compound is capable of inhibiting both of the blood coagulation reactions in the primary hemostasis stage in which platelets are involved and in the coagulation thrombus formation stage in which blood coagulation factors are involved in a state retaining the anticoagulant activity.

MEANS FOR SOLVING THE PROBLEMS

In order to solve the above problems, the present inventors have intensively studied to find out a medical material whose surface is immobilized a hydrophilic polymer compound in which a specific compound having an antithrombin activity and copolymer inhibiting the adhesion of platelets are bound exhibits a significant anticoagulant activity and the hydrophilic polymer compound is firmly immobilized to the surface of the medical material.

Accordingly, the present invention provides a medical material comprising a hydrophilic polymer compound immobilized on the surface thereof, said hydrophilic polymer compound in which a compound represented by the general formula (I) and a copolymer of monomers selected from the group consisting of ethylene glycol, vinyl acetate, vinyl pyrrolidone, propylene glycol, vinyl alcohol and siloxane are bound:

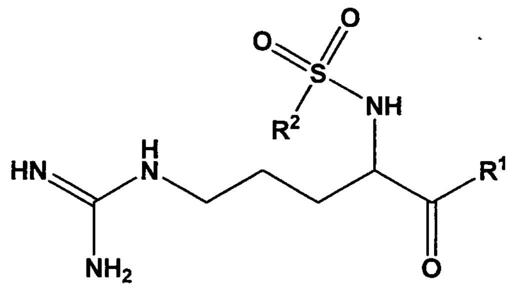

··· (I)

[wherein $R^1$ represents a (2R,4R)-4-alkyl-2-carboxypiperidino group; $R^2$ represents a phenyl group or a fused polycyclic compound group, the fused polycyclic compound group being optionally substituted with a lower alkyl group, a lower alkoxy group or an amino group which is substituted with a lower alkyl group].

The above copolymer is preferably a polyether-modified silicone.

The above compound represented by the general formula (I) is preferably (2R,4R)-4-methyl-1-((2S)-2-{[(3RS)-3-methyl-1,2,3,4-tetrahydroquinolin-8-yl]sulfonyl} amino-5-guanidinopentanoyl)piperidine-2-carboxylic acid.

Examples of materials of the above medical material include cellulose, cellulose acetate, polycarbonate, polysulfone (hereinafter referred to as "PSf"), polyether sulfone, polymethacrylate such as poly(methyl methacrylate) (hereinafter referred to as "PMMA"), polyacrylate, polyamide, polyvinylidene fluoride, polyvinyl chloride, polyacrylonitrile, polyester, polyurethane, polystyrene, polyethylene, polypropylene, polymethylpentene, polyimide and polytetrafluoroethylene. Preferred is polyester, polyurethane, polystyrene, PMMA, polyvinyl chloride, polytetrafluoroethylene, or PSf.

Examples of the above medical material include implantable artificial organs, synthetic blood vessels, catheters, stents, blood bags, blood circuits, artificial lungs, artificial hearts and lungs, organ adhesion preventive films, contact lenses, intraocular lenses, surgical auxiliary instruments and separation membranes such as hollow fiber membranes or adsorbents that are built in modules for biological component separation or modules for hemocatharsis. Preferred are hollow fiber membranes.

Further, the present invention also provides a module for hemocatharsis that is filled with a hollow fiber membrane comprising the above hydrophilic polymer compound immobilized on the surface thereof.

EFFECT OF THE INVENTION

According to the present invention, a medical material having a hydrophilic polymer compound firmly immobilized on the surface thereof, which compound significantly inhibits both of the blood coagulation reactions in the primary hemostasis stage in which platelets are involved and in the coagulation thrombus formation stage in which blood coagulation factors are involved in a state retaining such an anticoagulant activity can be provided.

BRIEF DESCRIPTION OF THE DRAWINGS

Figure 1 is a schematic view showing a mini-module prepared in the examples.

Figure 2 is a schematic view showing a closed circuit used in an *in vitro* blood circulation test.

Figure 3 is a schematic view showing a human blood plasma circulation circuit used in measurement of the amount of hydrophilic polymer compound dissolved out.

Figure 4 is a figure showing the results of *in vitro* blood circulation test using a PSf and PMMA hollow fiber membrane mini-module in conditions with no anticoagulants being added.

MODE FOR CARRYING OUT THE INVENTION

Unless otherwise specified, the terms used herein have the following definitions:

The term "hydrophilic" in the term "hydrophilic polymer compound" immobilized on the medical material of the present invention herein means that a compound is water-soluble, or even if a compound is not water-soluble, the compound interacts with water molecules by electrostatic interaction or hydrogen bond.

Examples of "copolymer of monomers selected from the group consisting of: ethylene glycol, vinyl acetate, vinyl pyrrolidone, propylene glycol, vinyl alcohol, and siloxane" (hereinafter referred to as "anti-platelet adhesion copolymer") include polymeric compounds composed of polyvinyl alcohol, polyvinyl pyrrolidone, polyethylene glycols, polypropylene glycol, polyether and polysiloxane or copolymers or graft materials of monomers of the polymeric compounds with other monomers. Preferred is a polymeric compound composed of highly hydrophilic polyether and polysiloxane or copolymer of partially saponified polyvinyl alcohol or vinyl pyrrolidone with vinyl acetate.

Examples of "polymer compound composed of polyether and polysiloxane" include copolymers, polymer complexes or polymer blend products of polyether and polysiloxane. The copolymer of polyether and polysiloxane is composed of polyether units and polysiloxane units, and the copolymer form thereof may be any of random copolymer, block copolymer or graft copolymer. Among these, polyether-modified silicone which is highly hydrophilic is preferred.

Examples of "polyether" include structures originated from polyethylene oxide or polypropylene oxide. "Polyether" herein refers to a structure represented by the general formula (II) ($R^3$ represents an alkyl group having 6 carbon atoms or less), and a "structure originated from polypropylene glycol" which is one of the examples of polyether refers to a structure represented by the general formula (III).

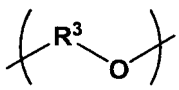

··· (II)

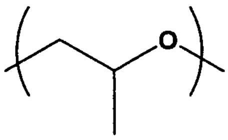

··· (III)

The term "polyether-modified silicone" refers to a silicone in which polyether units are bound to side chains of the silicone chain, and may be a polyether-modified silicone that is further amino-modified or carboxy-modified.

In cases where an anti-platelet adhesion copolymer is partially saponified polyvinyl alcohol, the degree of saponification thereof is preferably 50 to less than 100 mol% from the viewpoint of attaining suitable ease of handling or hydrophilicity, more preferably 74 to 99.9 mol%, and still more preferably 78 to 95 mol%. A "degree of saponification" herein refers to a numerical value calculated by the equation 1.

$$\text{Degree of saponification} = m/(n + m) \times 100 \cdots\cdots \text{Equation 1}$$

m: the number of structures represented by the general formula (IV) in polyvinyl alcohol

n: the number of structures represented by the general formula (V) in polyvinyl alcohol

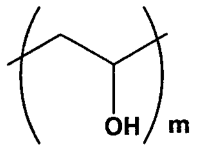

··· (IV)

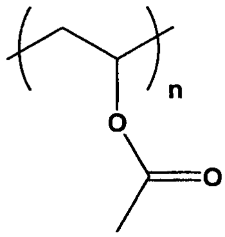

··· (V)

In cases where an anti-platelet adhesion copolymer is a copolymer of vinyl pyrrolidone and vinyl acetate, vinyl pyrrolidone units preferably account for 50 unit mol% or more, and more preferably 60 unit mol% or more, from the viewpoint of attaining suitable ease of handling or hydrophilicity. On the other hand, from the viewpoint of attaining a suitable amount of immobilization to a medical material, vinyl pyrrolidone units preferably account for less than 100 unit mol%. Note that the percentage of the vinyl pyrrolidone units in the copolymer of vinyl pyrrolidone and vinyl acetate (unit mol%) can be calculated by subjecting the copolymer to $^1$H-NMR measurement (solvent: $CDCl_3$).

The amount of adsorption of an anti-platelet adhesion copolymer on the surface of a medical material is preferably 0.1 pg/mm$^2$ or more, more preferably 1 pg/mm$^2$ or more, and still more preferably 10 pg/mm$^2$ or more.

The above amount of adsorption is measured by the following method: First, an untreated sensor chip (Sensor Chip Au; GE Healthcare) is pretreated (with distilled water at 25°C, at a flow rate of 20 μl/min, for 10 minutes) using a surface plasmon resonance system (hereinafter referred to as "SPR") (BIACORE 3000; GE Healthcare), and a signal value thereof (RU: resonance unit) is measured.

A material of medical material, that is, a material to be immobilized is dissolved in a solvent to prepare a 0.5% by weight solution of material to be immobilized. One drop of the solution of material to be immobilized is dropped onto the center of a gold film part of a pretreated sensor chip that has been installed in a spin coater and is immediately rotated at 3,000 rpm for 1 minute at room temperature to coat the sensor chip with the material to be immobilized.

After confirming that no droplets are present on the sensor chip, the sensor chip is washed with distilled water using SPR (at 25°C, at a flow rate of 20 μl/min, for 10 minutes), further washed three times with 0.025 wt % Triton-X100 solution (at 25°C, at a flow rate of 20 μl/min, for 1 minute), and then the signal value at 10 minutes after completion of the washing is measured.

Among the sensor chips obtained as described above, ones whose signal value difference before and after spin coat was within a range from 3,000 to 8,000 were selected, then washed with distilled water (at 25°C, at a flow rate of 20 μl/min, for 10 minutes), and further washed three times with 0.025 wt % Triton-X100 solution (at 25°C, at a flow rate of 20 μl/min, for 1 minute).

Ten minutes after completion of the washing, an aqueous solution of a hydrophilic polymer compound to be adsorbed to a medical material (concentration: 100 μg/ml) is injected (at 25°C, at a flow rate of 20 μl/min, for 1 minute), and washed with distilled water (at 25°C, at a flow rate of 20 μl/min, for 3 minutes). The difference between the signal value immediately before the injection (hereinafter referred to as "signal value A") and the signal value at 3 minutes after completion of the injection (hereinafter referred to as "signal value B") is determined, which is converted as 1 RU = 1 pg/mm$^2$.

Subsequently, the sensor chip is washed with distilled water (at 25°C, at a flow rate of 20 μl/min, for 2 minutes), further washed three times with 0.025 wt % Triton-X100 solution (at 25°C, at a flow rate of 20 μl/min, for 1 minute), and then the aqueous solution of the hydrophilic polymer compound to be adsorbed (concentration: 100 μg/ml) is again injected (at 25°C, at a flow rate of 20 μl/min, for 1 minute). Thereafter, the same operation is repeated to determine a signal difference (difference between signal value A and signal value B) for five times in total, and the mean value is regarded as the amount of adsorption of anti-platelet adhesion copolymer to the medical material.

As a "compound represented by the general formula (I)" which is a compound having a compound having a guanidine structure, (2R,4R)-4-methyl-1-((2S)-2-{[(3RS)-3-methyl-1,2,3,4-tetrahydroquinolin-8-yl]sulfonyl}amino-5-guanidinopentanoyl)piperidine-2-carboxylic acid (hereinafter referred to as "argatroban") is for example preferred. Argatroban synthesized in 1978 is a medicinal compound having a selective antithrombin activity of arginine derivatives. The phrase "having an antithrombin activity" herein means that a binding affinity to thrombin is high, and examples of indexes for evaluating the antithrombin activity of a compound include an inhibition constant (hereinafter referred to as

"Ki") which is calculated from Lineweaver-Burk plot based on the absorbance value of solution to be tested. Lower Ki indicates higher binding affinity to thrombin and higher antithrombin activity. Ki is preferably 10 μM or less, more preferably 1 μM or less, and still more preferably 500 nM or less. The compound having an antithrombin activity can be used solely or two or more thereof can be combined to use.

Examples of a method of immobilizing the above hydrophilic polymer compound onto the surface of a medical material include a method comprising bringing a surface treatment agent containing the above hydrophilicity compound as a active component into contact with the medical material and irradiating thereto with radiation. Note that, as the type of the irradiating radiation, an electron beam or γ-ray is preferred. In cases where the method comprising irradiating the radiation is difficult, a method comprising spraying or spreading the above hydrophilicity compound dissolved or dispersed in an organic solvent such as ethanol or methanol and onto the surface of the medical material and drying can be for example employed.

EXAMPLES

The present invention will be now described in detail below by way of the examples. However, the present invention is by no means limited thereto.

(Example 1)

1. Binding between Amino·Polyether-Modified Silicone and Argatroban:

Argatroban in an amount of 5 mmol was placed in a recovery flask, and 10 mL of anhydrous dimethylformamide (hereinafter referred to as "anhydrous DMF") was added thereto to dissolve argatroban. Thereafter, 10 mL of 4N hydrochloric acid/1,4-dioxane (Toyo Kasei Co., Ltd.) was added dropwise while cooling the recovery flask and the resulting mixture was stirred for 1 hour. Next, the solvent was evaporated with a rotary evaporator and the resultant was further dried overnight in a vacuum dryer, and added with 25 mL of anhydrous DMF to obtain argatroban hydrochloride/anhydrous DMF solution.

Argatroban hydrochloride/anhydrous DMF solution in an amount shown in Table 1 was placed in a two-necked flask, and dicyclohexylcarbodiimide (hereinafter referred to as "DCC")/anhydrous DMF solution and 4-hydroxybenzotriazole , (hereinafter referred to as "HOBt")/anhydrous DMF solution were each added thereto with ice cooling and stirring. A polyether-modified silicone (X-22-3939A; Shin-Etsu Chemical Co., Ltd.) was further added and the resulting mixture was allowed to react at room temperature for 3 days. Next, the reaction solution was placed in a dialysis tube (Spectra/Por RC, Pore 6, MWCO=1,000), and dialyzed for 3 days against distilled water with a volume of more than 10 times while appropriately replacing the distilled water. The reaction solution after the dialysis was filtered, and the solvent in the filtrate was evaporated with a rotary evaporator, followed by drying the resultant overnight in a vacuum dryer to obtain a hydrophilic polymer compound (hereinafter referred to as "Example 1 compound").

2. Measurement of Antithrombin Activity of Example 1 compound

ECA-T kit (HaemoSys) was used for the measurement. To 100 μL of the Example 1 compound, 900 μL of distilled water was added to prepare an aqueous Example 1 compound solution. The aqueous Example 1 compound solution in an amount of 30 μL was sampled and mixed with 100 μL of ECA prothrombin buffer and 25 μL of ECA-T substrate. After incubated at 37°C for 60 seconds, the mixture was set in an apparatus (COATRON M1(code 80 800 000); Production); and 50 μL of ECA ecarin reagent was added thereto to then carry out the measurement.

A mixture of 20 μL of argatroban solution prepared to an arbitrary concentration using an ethanol/hydrochloric acid (volume ratio: 4/1) mixed solvent and 80 μL of human blood plasma, or a mixture of 20 μL of blank distilled water and 80 μL of human blood plasma was each subjected to the measurement using the ECA-T kit in place of the above aqueous Example 1 compound solution, and a calibration curve was prepared from the results. The concentration of 1494.3 ppm by weight in terms of argatroban of the aqueous Example 1 compound solution calculated from the calibration curve was defined as a value indicating the antithrombin activity of the aqueous Example 1 compound solution.

3. Measurement of Thrombin Inhibition Constant of Example 1 Compound

A bovine thrombin solution (Ito Life Sciences, Inc.) 10,000 U was dissolved in 1 mL of physiological saline to prepare an aqueous bovine thrombin solution.

S-2238 stock solution (Sekisui Medical Co., Ltd.) 25 mg was dissolved in 40 mL of distilled water to prepare an aqueous S-2238 stock solution.

The aqueous bovine thrombin solution, aqueous S-2238 stock solution and the above aqueous Example 1

compound solution were each diluted by using dilution buffer solution (0.05 M Tris, 0.1 M NaCl, 1 mg/mL of bovine serum albumin (BSA), pH 7.4).

To a 96-well plate, 100 μL of diluted solution of the aqueous S-2238 stock solution and 50 μL of diluted solution of the aqueous Example 1 compound solution were dispensed, sealed and then warmed in a thermostat dryer set at 37°C for 30 minutes. Next, 50 μL of the diluted solution of the aqueous bovine thrombin solution which was heated at 37°C for 30 minutes was further dispensed thereto, and absorbance of the resulting mixture was immediately measured with a microplate reader (measurement wavelength: 405 nm, reference wavelength: 595 nm).

Immediately after completion of the first absorbance measurement, the second absorbance measurement was carried out. The third or later absorbance measurements were carried out at 4, 6, 8, 10, 12, 14, 16, 18, and 20 minutes, respectively, after the diluted solution of the aqueous bovine thrombin solution was dispensed. Ki was calculated from each of the obtained absorbance values by using Lineweaver-Burk plot. The Ki of the Example 1 compound was 11 nM.

The Ki of the polyether-modified silicone (X-22-3939A) was also calculated in the same manner, but the Ki of the polyether-modified silicone without an antithrombin activity was the same as that of the blank, as expected.

Further, Ki was also calculated for argatroban in the same manner, and the Ki thereof was 39 nM which was a three times or more higher value, as compared with the Ki of the Example 1 compound.

From these results, it became apparent that the above hydrophilic polymer compound has an extremely high binding affinity to thrombin, and can give a prominent antithrombin activity that is much higher than argatroban, which is known to have an antithrombin activity, to medical materials including hollow fiber membranes.

(Examples 2 to 13)

Examples 2 to 13 compounds were obtained, respectively, in the same manner as described in Example 1 except that the molar ratios of DCC, HOBt and polyether-modified silicone (X-22-3939A) to argatroban hydrochloride and the volume ratio of anhydrous DMF to polyether-modified silicone were altered and the antithrombin activity thereof was measured. The molar ratios of DCC, HOBt and polyether-modified silicone (X-22-3939A) to argatroban hydrochloride and the measurement results of the antithrombin activity of each of the Examples 2 to 13 compounds are shown in Table 1.

[Table 1]

| Compound | Molar ratio to 1.00 mol of argatroban hydrochloride | | | Volume ratio of anhydrous DMF to 1 volume of polyether-modified silicone | Concentration in terms of argatroban (ppm by weight) |
|---|---|---|---|---|---|
| | DCC | HOBt | X-22-3939A | | |
| Example 1 | 1.07 | 1.06 | 0.060 | - | 1494.3 |
| Example 2 | 1.04 | 1.04 | 0.060 | - | 831.2 |
| Example 3 | 0.20 | 0.20 | 0.060 | 1.4 | 6610.7 |
| Example 4 | 0.20 | 0.20 | 0.030 | 3.9 | 8393.3 |
| Example 5 | 1.29 | 1.27 | 0.493 | 1.8 | 505.3 |
| Example 6 | 1.29 | 1.27 | 0.203 | 4.3 | 771.7 |
| Example 7 | 1.29 | 1.27 | 0.101 | 8.6 | 606.7 |
| Example 8 | 1.29 | 1.27 | 0.067 | 13.0 | 441.7 |
| Example 9 | 1.29 | 1.27 | 0.049 | 17.6 | 436.7 |
| Example 10 | 1.29 | 1.27 | 0.020 | 42.9 | 738.9 |
| Example 11 | 1.29 | 1.27 | 0.010 | 88.2 | 895.0 |
| Example 12 | 1.00 | 1.00 | 0.060 | - | 6000.0 |
| Example 13 | 1.00 | 1.00 | 0.060 | 40.0 | 5999.4 |

The antithrombin activity of the polyether-modified silicone (X-22-3939A) was also measured and the measured value was the same as that of the blank distilled water. It was confirmed that the polyether-modified silicone itself does not have the antithrombin activity.

(Preparation of PMMA Hollow Fiber)

Five parts by weight of isotactic-PMMA and 20 parts by weight of syndiotactic-PMMA were added to 75 parts by weight of dimethylsulfoxide, and the resulting mixture was stirred at 110°C for 8 hours to obtain a membrane-forming liquid. This membrane-forming liquid was extruded from an orifice type bicylindrical mouthpiece and passed 300 mm in air, and then the extruded material was introduced into a solidification bath containing 100% water to obtain PMMA hollow fibers having an inner diameter of 0.2 mm and a membrane thickness of 0.03 mm. Note that, as the gas injected into the inside of the fiber, dry nitrogen was used.

(Preparation of PSf hollow fiber)

Eighteen parts by weight of PSf (Udel manufactured by Solvay (registered trademark) P-3500) and 9 parts by weight of PVP (K30 manufactured by BASF) were added to a mixed solvent of 72 parts by weight of DMAc and 1 part by weight of water, and the resulting mixture was stirred at 90°C for 14 hours to obtain a membrane-forming liquid. Also, a core liquid composed of 58 parts by weight of DMAc and 42 parts by weight of water was prepared. Using an orifice type bicylindrical mouthpiece whose cyclic slit part had an outer diameter of 0.3 mm and an inner diameter of 0.2 mm, the membrane-forming liquid and core liquid were extruded from the outside tube and inside tube, respectively, and introduced into a solidification bath containing 100% water that was located 350 mm away from the mouthpiece to obtain PSf hollow fibers.

(Preparation of PMMA Hollow Fiber Membrane Mini-Module)

A module case having an inner diameter of 10 mm and a length of 120 mm that, similarly to general hollow fiber-type dialyzers, had two ports communicating to the inside of the hollow fibers (blood port) and two ports communicating to the outside of the hollow fibers (dialysate port) was prepared.

Fifty of the above PMMA hollow fibers were bundled to form a PMMA hollow fiber membrane, and both ends of the PMMA hollow fiber membrane were fixed to the above module case using an epoxy-based potting agent with attention not to clog the hollow portion of the PMMA hollow fiber membrane. Thereafter, the PMMA hollow fiber membrane and the inside of the module case were washed with distilled water to obtain the mini-module 6 as shown in Figure 1.

Bis-Tris (Dojindo Laboratories) and sodium chloride were dissolved in ultrapure water so as to attain a final concentration of 0.25M and 0.5M, respectively, and the pH of the resulting solution was adjusted to 5 by adding 6N hydrochloric acid dropwise to prepare Bis-Tris buffer solution having 5 times concentration.

Distilled water remaining at the side contacting the blood (the inner side of the PMMA hollow fiber membrane) and the side not contacting the blood (the outer side of the PMMA hollow fiber membrane) of the prepared mini-module 6 was removed with compressed air. Next, the aqueous Example 1 compound solution of a concentration of 10,000 ppm by weight in terms of argatroban, propylene glycol, Bis-Tris buffer solution having 5 times concentration, and distilled water were mixed at a volume ratio of 4/3/2/1 to obtain a filling solution.

The above filling solution (400 $\mu$L) was filled only at the side contacting the blood of the mini-module 6 using a syringe. Thereafter, the filling solution was removed with compressed air, and the mini-module 6 in which all of the blood ports 1a and 1b and the dialysate ports 2a and 2b were tightly capped was irradiated with $\gamma$-ray at a absorbed dose of 25 kGy for about 3 hours.

The PMMA hollow fiber membrane 4 and the inner side of the mini-module 6 were washed by passing 0.025% by weight aqueous poly(oxyethylene)octyl phenyl ether solution into the PMMA hollow fiber membranes 4 and the inner side of the mini-module 6 at a flow rate of 10 mL/min for 8 hours using the peristaltic pump 8. Thereafter, distilled water and physiological saline were flown both at a flow rate of 10 mL/min for 30 minutes to carry out further washing to obtain a mini-module on which the Example 1 compound was immobilized (hereinafter referred to as "PMMA hollow fiber membrane mini-module 1").

The aqueous Example 1 compound solution of a concentration of 10,000 ppm by weight in terms of argatroban, propylene glycol, Bis-Tris buffer solution having 5 times concentration, and distilled water were mixed at a volume ratio shown in Table 2 to obtain each filling solution. Each of the mini-modules on which the Example 1 compound was immobilized ("PMMA hollow fiber membrane mini-module 2" to "PMMA hollow fiber membrane mini-module 4") was prepared by carrying out the same procedure as described above except that each of the prepared filling solutions was used.

[Table 2]

| Mini-module | | Aqueous Example 1 compound solution of a concentration of 10,000 ppm by weight in terms of argatroban | Propylene glycol | Bis-Tris buffer solution having 5 times concentration | Distilled water |
|---|---|---|---|---|---|
| PMMA | 1 | 4 | 3 | 2 | 1 |
| | 2 | 0.1 | 3 | 2 | 4.9 |
| | 3 | 1 | 3 | 2 | 4 |
| | 4 | 5 | 3 | 2 | 0 |

(Preparation of PSf Hollow Fiber Membrane Mini-Module)

A mini-modules on which the Example 1 compound was immobilized (hereinafter referred to as "PSf hollow fiber membrane mini-module 2") was prepared by carrying out the same procedure as described above except that PMMA hollow fibers were replaced with PSf hollow fibers.

Distilled water remaining at the side contacting the blood (the inner side of the PSf hollow fiber membrane) and the side not contacting the blood (the outer side of the PSf hollow fiber membrane) of the separately prepared mini-module 6 was removed with compressed air. Next, the aqueous Example 1 compound solution of a concentration of 10,000 ppm by weight in terms of argatroban, propylene glycol, Bis-Tris buffer solution having 5 times concentration, and distilled water were mixed at a volume ratio of 4/3/2/1 to obtain a filling solution.

The above filling solution (400 $\mu$L) was filled only at the side contacting the blood of the mini-module 6 using a syringe. Thereafter, the filling solution was removed with compressed air, and the mini-module 6 in which all of the blood ports 1a and 1b and the dialysate ports 2a and 2b were tightly capped was irradiated with $\gamma$-ray at a absorbed dose of 25 kGy for about 3 hours.

The PSf hollow fiber membrane 4 and the inner side of the mini-module 6 were washed by passing 0.025% by weight aqueous poly(oxyethylene)octyl phenyl ether solution into the PSf hollow fiber membranes 4 and the inner side of the mini-module 6 at a flow rate of 10 mL/min for 8 hours using the peristaltic pump 8. Thereafter, distilled water and physiological saline were flown both at a flow rate of 10 mL/min for 30 minutes to carry out further washing to obtain a mini-module on which the Example 1 compound was immobilized (hereinafter referred to as "PSf hollow fiber membrane mini-module 1").

The aqueous Example 1 compound solution in a concentration of 10,000 ppm by weight in terms of argatroban, propylene glycol, and Bis-Tris buffer solution having 5 times concentration were mixed at a volume ratio shown in Table 3 to obtain each filling solution. Each of the mini-modules on which the Example 1 compound was immobilized ("PSf hollow fiber membrane mini-module 2" to "PSf hollow fiber membrane mini-module 5") was prepared by carrying out the same procedure as described above except that each of the prepared filling solutions was used.

[Table 3]

| Mini-module | | Aqueous Example 1 compound solution of a concentration of 10,000 ppm by weight in terms of argatroban | Propylene glycol | Bis-Tris buffer solution having 5 times concentration | Distilled water |
|---|---|---|---|---|---|
| PSf | 1 | 4 | 3 | 2 | 1 |
| | 2 | 0.1 | 3 | 2 | 4.9 |
| | 3 | 1 | 3 | 2 | 4 |
| | 4 | 2 | 3 | 2 | 3 |
| | 5 | 5 | 3 | 2 | 0 |

A mini-module in which polyether-modified silicone was immobilized (hereinafter referred to as "Comparative Example 1 mini-module") was obtained by carrying out the same procedure as described above except that polyether-modified silicone (X-22-3939A) was used in place of the aqueous Example 1 compound solution of a concentration of 10,000 ppm by weight in terms of argatroban.

(*In Vitro* Blood Circulation Test)

Blood provided by a volunteer and citric acid was mixed at a volume ratio of 9/1 to obtain blood supplemented with citric acid. Calcicol in an amount of 43.6 μL was added, as a procoagulant, to 1 mL of the blood supplemented with citric acid to obtain a test blood.

Silicone tubes 7a and 7b were connected to the PMMA hollow fiber membrane mini-module 1 and the peristaltic pump 8 was placed in the middle of the silicone tube 7b. The test blood was passed at a flow rate of 0.9 mL/min for 5 seconds from the silicone tube 7a connected to the blood port 1a, and the test blood flown out from the blood port 1b was discarded from the silicone tube 7b to remove bubbles in the inner side of the PMMA hollow fiber membrane. Subsequently, the silicone tubes 7a and 7b were connected at the inclusion part 9 to form a closed circuit shown in Figure 2.

The circulation of the test blood was started at a flow rate of 0.9 mL/min to measure duration time of circulation until the silicone tubes 7a or 7b came off the inclusion part 9 due to an increased inner pressure in the circuit caused by coagulation thrombus formed in the circuit. The duration time of circulation in the case of using the PMMA hollow fiber membrane mini-module 1 was 35 minutes. Further when evaluated under the same condition, the duration time of circulation in the case of using the PSf hollow fiber membrane mini-module 1 was 41 minutes.

Figure 4 shows the results of the *in vitro* blood circulation test that was carried out as described above using, in place of the PMMA hollow fiber membrane mini-module 1, each of the PMMA hollow fiber membrane mini-modules 2 to 4 and PSf hollow fiber membrane mini-modules 2 to 5. In the PMMA hollow fiber membrane mini-module, the duration time of circulation reached a plateau once the volume ratio of the Example 1 compound solution in the filling solution exceeded a certain value. In contrast, this tendency was not observed in the PSf hollow fiber membrane mini-module and the duration time of circulation could be continuously extended as appropriate.

The mini-module 6 in which no compounds were immobilized on the PMMA hollow fiber membrane (hereinafter referred to as "Comparative Example 2 mini-module") was prepared to carry out the same blood circulation test as described above. The duration time of circulation in this case was 20 minutes which was half or less than that in the case of using the PMMA hollow fiber membrane mini-module or PSf hollow fiber membrane mini-module. From these results, it became apparent that the above hydrophilic polymer compound exhibited an excellent anticoagulant activity for the medical material immobilized therewith.

Note that, when the same blood circulation test was carried out as described above using the Comparative Example 1 mini-module, the duration time of circulation was 20 minutes, which was the same as the duration of circulation in the case of using the Comparative Example 2 mini-module in which no compounds were immobilized on the PMMA hollow fiber membrane.

(Measurement of Amount of Example 1 Compound Dissolved Out)

The silicone tube 7b having an inner diameter of 0.8 mm and a length of 520 mm was connected to the blood port 1b of the separately prepared Example 1 mini-module, and the peristaltic pump 8 was placed in the middle of the silicone tube 7b. The silicone tube 7a having an inner diameter of 0.8 mm and a length of 160 mm was connected to the blood port 1a. Thereafter, the other end of each of the silicone tubes 7a and 7b was inserted in the polystyrene round tube (Code: 352054; BECTON DICKINSON) 10 containing 5 mL of human plasma to prepare a circulating circuit shown

in Figure 3.

Human plasma was circulated at a flow rate of 0.5 mL/min for 4 hours using the peristaltic pump 8, the concentration of the Example 1 compound in human plasma in the polystyrene round tube 10 was measured using ECA-T kit. However, the concentration of the Example 1 compound in human plasma after the circulation was below the limit of detection of ECA-T kit, and it was not confirmed that the Example 1 compound dissolved out from the PMMA hollow fiber membrane mini-module. This result shows that the above hydrophilic polymer compound can be firmly immobilized to medical materials including a hollow fiber membrane.

(Evaluation of Adsorption Amount of Anti-Platelet Adhesion Copolymer)

As a copolymer between vinylpyrrolidone and vinyl acetate (hereinafter referred to as "VA copolymer"), which copolymer composes the above hydrophilic polymer compound and was one of the anti-platelet adhesion copolymers, PVP (K-90), VA73, VA64, VA55 and VA37 (all of which were from BASF) were provided. Similarly, as a partially saponified polyvinyl alcohol which was one of the anti-platelet adhesion copolymers, PVA217, PVA417 and PVA205c (all of which were from Kuraray Co., Ltd.) were provided. Further, as a polyether-modified silicone, F114, F244, F303, F3031, F348, F350s, F502, F506 and X-22-3939A (all of which were from Shin-Etsu Silicone) were provided. Note that the provided VA copolymer, partially saponified polyvinyl alcohol and polyether-modified silicone were all diluted with distilled water to prepare an aqueous solution of 10,000 ppm by weight.

Meanwhile, as a polymer compound composing the above hydrophilic polymer compound, which was not included in the anti-platelet adhesion copolymer, PEG2000, PEG4000, PEG6000 and PEG20000 (all of which were from Nacalai Tesque, Inc.), and PEG methyl ether (PEG-em) and PEG dimethyl ether (PEG-dm) (both were from Sigma-Aldrich) were provided for comparison. Note that the provided polymer compounds were all diluted with distilled water to prepare an aqueous solution of 10,000 ppm by weight.

As a 0.5% by weight solution of a material to be immobilized for immobilizing an anti-platelet adhesion copolymer, PMMA (weight average molecular weight: 93000, Sigma-Aldrich)/toluene solution, polyurethane/dimethylacetamide solution, PSf (Udel manufactured by Solvay (registered trademark) P-3500)/dimethylacetamide solution, polyvinyl chloride (weight average molecular weight: 80,000; Sigma-Aldrich)/tetrahydrofuran solution, polystyrene (Wako)/chloroform solution and polycarbonate (weight average molecular weight: 20,000; Teijin Limited)/chloroform solution were each prepared.

The adsorption amount of various anti-platelet adhesion copolymers for each of the materials to be immobilized was measured. The results are shown in Figure 4.

[Table 4]

| Signal value B - Signal value A [pg/mm$^2$] | | | | | | | |
|---|---|---|---|---|---|---|---|
| | | Adsorbent Material | | | | | |
| | | PMMA | PSf | Poly-urethane | Polyvinyl chloride | Poly-styrene | Poly-carbonate |
| Anti-platelet adhesion copolymer | PVPK 90 | 789 | - | - | - | - | - |
| | VA37 | 2760 | - | - | - | - | - |
| | VA55 | 472 | - | - | - | - | - |
| | VA64 | 920 | - | - | - | - | - |
| | VA73 | 426 | - | - | - | - | - |
| | PVA 217 | 2529 | 2886 | 1635 | 2468 | 2777 | 2356 |
| | PVA 417 | 2475 | 2742 | 1911 | 2330 | 2662 | 2346 |
| | PVA 205c | 2223 | 2130 | 1411 | 1796 | 1989 | 1819 |
| | F114 | 1003 | 844 | 514 | 739 | 621 | 756 |
| | F244 | 1639 | 1272 | 1144 | 1118 | 1052 | 1243 |
| | F303 | 1268 | 1156 | 1604 | 1037 | - | 1374 |
| | F3031 | 947 | 559 | 614 | 418 | 339 | 536 |
| | F348 | 875 | 784 | 756 | 608 | 283 | 800 |
| | F350s | 751 | 657 | 674 | 544 | 275 | 591 |
| | F502 | 827 | 657 | 696 | 385 | 197 | 482 |
| | F506 | 691 | 308 | 437 | 167 | 43 | 279 |
| | X-22-3939A | 1182 | 910 | 1204 | 695 | 924 | 1424 |
| | PEG 2000 | 2 | - | - | - | - | - |
| | PEG 4000 | 2 | - | - | - | - | - |
| | PEG 6000 | 5 | - | - | - | - | - |
| | PEG 20000 | 113 | - | - | - | - | - |
| | PEG -me | 5 | - | - | - | - | - |
| | PEG -dm | 67 | - | - | - | - | - |

From the results of Table 4, it became apparent that the anti-platelet adhesion copolymer composing the above hydrophilic polymer compound was not limited to the polyether-modified silicone (X-22-3939A) and could be firmly immobilized to medical materials including a hollow fiber membrane.

(Evaluation of Anti-Platelet Adhesion Ability)

The separately prepared module case of the PMMA hollow fiber membrane mini-module was cut with an ultrasonic cutter to take out the PMMA hollow fiber membrane (hereinafter referred to as "Example 1 hollow fiber membrane") on which the Example 1 compound was immobilized.

A double-stick tape was adhered to one surface of a circular film made of polyethylene terephthalate having a diameter of 18 mm. The Example 1 hollow fiber membrane was fixed thereto, and the fixed PMMA hollow fiber membrane was cut into slivers of a semicylindrical shape to expose the inner surface of the PMMA hollow fiber membrane. The Example hollow fiber membrane fixed to the circular film was placed inside of a Falcon (registered trademark) cylindrical tube (18 mmΦ, NO. 2051) that had cut into a cylindrical shape, and the gap between the cylindrical tube and circular film was sealed with Parafilm. Thereafter, the cylindrical tube was filled with physiological saline.

Venous blood immediately after collected from volunteers was placed in a blood collection tube in which heparin had been collected in advance, and the resulting mixture was mixed by inverting the tube to prepare blood supplemented with heparin. The concentration of the blood supplemented with heparin was adjusted to be 50 U/mL.

After discarding the physiological saline in the above cylindrical tube, 1.0 mL of the blood supplemented with heparin was placed thereto, and the cylindrical tube was shaken at 37°C for 1 hour. Thereafter, the Example 1 hollow fiber membrane in the above cylindrical tube was washed with 10 mL of physiological saline, and then blood components were fixed by adding physiological saline containing 2.5% by volume glutaraldehyde, followed by further washing the membranes with distilled water. Thereafter, the circular film on which the Example 1 hollow fiber membrane was fixed was removed from the above cylindrical tube, and the circular film on which the Example hollow fiber membrane was fixed was dried under reduced pressure at normal temperature at an absolute pressure of 0.5 Torr for 12 hours.

The circular film dried under reduced pressure on which the Example 1 hollow fiber membrane was fixed was adhered to the stage of a scanning electron microscope with a double-stick tape, and a platinum/palladium thin film was then formed on the surface of the Example hollow fiber membranes by sputtering. The inner surface near the center in the longitudinal direction of the Example hollow fiber membrane, wherein a platinum/palladium thin film was formed on the surface thereof, was observed with a field emission scanning electron microscope (S800; manufactured by Hitachi, Ltd.) at a magnification of 1500×, and the number of adhered platelets in one visual field ($4.3\times10^3$ $\mu m^2$) was counted.

The integer value of the mean of the numbers of adhered platelets counted in different 5 visual fields was defmed as the number of adhered platelets (platelets/($4.3\times10^3 \mu m^2$)), and the number of the platelets adhered to the Example 1 hollow fiber membrane was 1.

On the other hand, the separately prepared module case of the Comparative Example 2 mini-module was cut with an ultrasonic cutter, and the hollow fiber membrane on which any compounds were not immobilized (hereinafter referred to as "Comparative Example 2 hollow fiber membrane") was taken out to confirm the number of adhered platelets as well. The number of the platelets adhered to the Comparative Example 2 hollow fiber membrane was 100 or more.

From these results, it became apparent that the above hydrophilic polymer compounds can impart a significant anti-platelet adhesion ability to medical materials including a hollow fiber membrane.

(Measurement of Whole Blood Clotting Time)

The blood collected from volunteers and citric acid were mixed at a volume ratio of 9/1 to prepare blood supplemented with citric acid.

To a cuvette (NON-ACTIVATED CLOTTING TEST KIT), 18 μL of physiological saline was placed, and 14.8 μL of calcicol was added thereto, followed by further adding 342 μL of blood supplemented with citric acid. Thereafter, the measurement using Sonoclot blood coagulation/platelet function analyzer (IMI Co., Ltd.) was carried out to define the obtained ACT ONSET value as whole blood clotting time. The whole blood clotting time of the blood collected by the volunteers was 545 seconds.

When the same measurement were carried out using, in place of physiological saline, each of 2, 10 and 20 μM of argatroban solutions (solvent was methanol/hydrochloric acid (volume ratio: 4/1)), the whole blood clotting times was 531, 746 and 849 seconds, respectively.

When the same measurements were carried out using, in place of physiological saline, each of the aqueous Example 1 compound solutions of 0.3, 1.3 and 2.5 μM, the whole blood clotting times was 527, 693 and 730 seconds, respectively.

(Example 14)

1. Binding between Vinyl Acetate-Vinyl Pyrrolidone Copolymer and Argatroban

To a screw vial, 14.9 g of tetrahydrofuran, 11.5 g of vinyl acetate, 10.8 g of N-vinylpyrrolidone, 0.028 g of 2-aminoethanethiol and 0.016 g of azobisisobutyronitrile were placed, and after sealing the screw vial, the resulting mixture was sonicated for 10 minutes. The screw vial was once unsealed, and the mixture was bubbled with argon gas for 10 minutes. The screw vial was again sealed, and then immersed in a hot water bath at 60°C under stirring for 1 hour and further in a hot water bath at 70°C for 6 hours to copolymerize vinyl acetate with vinyl pyrrolidone. To this reaction solution, 80 mL of methanol was added, and the resulting mixture was added to about 5 times amount of ether, followed by removing the supernatant. The washing procedure of newly adding ether and removing the supernatant was repeated three times, and the resultant was dried under reduced pressure to obtain a vinyl acetate-vinyl pyrrolidone copolymer. When the obtained vinyl acetate-vinyl pyrrolidone copolymer was measured by $^1$H-NMR (solvent: $CDCl_3$), a vinylpyrrolidone unit was 60.6 unit % by mole.

The obtained vinyl acetate-vinyl pyrrolidone copolymer, 3.58 g was dissolved in 20 mL of anhydrous DMF to prepare a vinyl acetate-vinyl pyrrolidone copolymer/anhydrous DMF solution. The entire volume of the prepared vinyl acetate-vinyl pyrrolidone copolymer/anhydrous DMF solution and 0.5 mL of argatroban hydrochloride/anhydrous DMF solution (0.49 M) were placed in a two-necked flask, and 0.5 mL of DCC/anhydrous DMF solution (1.04 M) and 0.5 mL of HOBt/anhydrous DMF solution (1.02 M) were each added thereto with ice cooling and stirring; and the resultant was allowed to react under a nitrogen atmosphere at room temperature for 3 days. Next, the reaction solution was placed in a dialysis tube (Spectra/Por RC, Pore 6, MWCO=1,000), and dialyzed for 3 days against distilled water with a volume of more than 10 times while appropriately replacing the distilled water. The reaction solution after the dialysis was filtered, and the solvent in the filtrate was evaporated with a rotary evaporator, followed by drying the resultant overnight in a vacuum dryer to obtain a hydrophilic polymer compound (hereinafter referred to as "Example 14 compound").

2. Measurement of Antithrombin Activity of Example 14 Compound

The antithrombin activity of the Example 14 compound/methanol solution (concentration: 20% by weight) was measured in the same manner as the measurement of the antithrombin activity of the Example 1 compound, and the calculated concentration of 104.1 ppm in terms of argatroban of the Example 14 compound/methanol solution was defined as a value indicating the antithrombin activity of the Example 14 compound/methanol solution.

(Example 15)

Argatroban in an amount of 44.8 mmol was placed in a recovery flask and 50 mL of anhydrous dimethylformamide (hereinafter referred to as "anhydrous DMF") was added thereto to dissolve argatroban under an Ar flow. Thereafter, the recovery flask was cooled in ice. Fifty milliliters of 4 N hydrochloric acid/1,4-dioxane (Toyo Kasei Co., Ltd.) were added dropwise and the resulting mixture was stirred at room temperature for 1 hour. Next, the solvent was evaporated using a rotary evaporator and subjected to azeotropic treatment with dehydrated toluene (Wako). The resultant was further dried in a vacuum dryer overnight; and anhydrous DMF was added to the obtained compound to make an argatroban hydrochloride/anhydrous DMF solution (1.0 M).

To a three-necked flask, 46 mL of argatroban hydrochloride/anhydrous DMF solution was added; and 57.8 mmol of HOBt and 20 mL of anhydrous DMF were added thereto with ice cooling and stirring. After the mixture was dissolved, 51.0 mmol of DCC was added. To 190 g of amino-polyether-modified silicone (X-22-3939A; Shin-Etsu Chemical Co., Ltd.) that was in advance dried under reduced pressure at 40°C for 5 hours, 760 g of anhydrous DMF was added and stirred. The anhydrous DMF solution in which argatroban hydrochloride, DCC, and HOBt had been dissolved was added to the amino-polyether-modified silicone/anhydrous DMF solution with ice cooling. Degassing and Ar substitution were repeated five times and then the mixture was stirred at room temperature for three days and allowed to react. Next, the reaction solution was placed in a dialysis tube (Spectra/Por RC, Pore 6, MWCO=15,000), and dialyzed for 7 days against distilled water with a volume of more than 100 times while appropriately replacing the distilled water. The reaction solution after the dialysis was filtered, and the solvent in the filtrate was evaporated with a rotary evaporator, followed by drying the resultant overnight in a vacuum dryer to obtain a bound substance (hereinafter referred to as "Example 15 bound substance").

(Measurement of Antithrombin Activity of Example 15 Bound Substance)

ECA-T kit (HaemoSys) was used for the measurement. To 10 mg of Example 15 bound substance, 1 ml of distilled water was added to prepare an aqueous Example 15 bound substance solution. The aqueous Example 15 bound substance solution in an amount of 30 μL was sampled and mixed with 100 μL of ECA prothrombin buffer and 25 μL of ECA-T substrate. After incubated at 37°C for 60 seconds, the mixture was set in an apparatus (COATRON M1(code 80 800 000); Production); and 50 μL of ECA ecarin reagent was added thereto to then carry out the measurement of antithrombin activity.

A mixture of 20 μL of argatroban solution prepared to an arbitrary concentration using an ethanol/hydrochloric

acid (volume ratio: 4/1) mixed solvent and 80 μL of human blood plasma, or a mixture of 20 μL of blank distilled water and 80 μL of human blood plasma was each subjected to the measurement using the ECA-T kit in place of the above aqueous Example 15 bound substance solution, and a calibration curve was prepared from the results. The concentration of 2.6 ppm by weight in terms of argatroban of the aqueous Example 15 bound substance solution calculated from the calibration curve was defined as a value indicating the antithrombin activity of the aqueous Example 15 bound substance solution.

From these results, it became apparent that the above hydrophilic polymer compound can prolong the whole blood clotting time as compared with argatroban which is known to have an antithrombin activity, even if the concentrations of the hydrophilic polymer compound are very low; and the hydrophilic polymer compound can impart an excellent anticoagulant activity to medical materials including a hollow fiber membrane filled in a module for hemocatharsis.

INDUSTRIAL APPLICABILITY

The present invention can be used as a medical material having an excellent anticoagulant activity in the field of medicine.

DESCRIPTION OF SYMBOLS

1a, 1b ··· Blood port; 2a, 2b ··· Dialysate port; 3 ··· Module case; 4 ··· Hollow fiber membrane; 5 ··· Potting agent; 6 ··· Mini-module; 7a, 7b ··· Silicone tube; 8 ··· Peristaltic pump; 9 ··· Inclusion part; 10 ··· Polystyrene round tube

## Claims

1. A medical material comprising a hydrophilic polymer compound immobilized on the surface thereof, said hydrophilic polymer compound being one in which
a compound represented by the following general formula (I) and
a copolymer of monomers selected from the group consisting of ethylene glycol, vinyl acetate, vinyl pyrrolidone, propylene glycol, vinyl alcohol, and siloxane are bound:

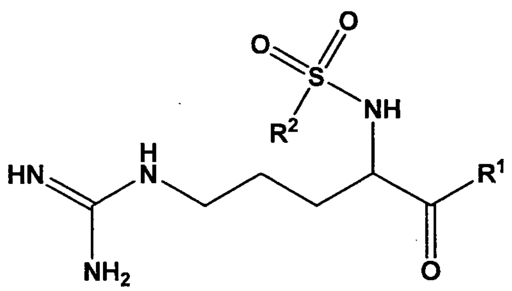

··· (I)

[wherein $R^1$ represents a (2R,4R)-4-alkyl-2-carboxypiperidino group; $R^2$ represents a phenyl group or a fused polycyclic compound group, the fused polycyclic compound group being optionally substituted with a lower alkyl group, a lower alkoxy group or an amino group which is substituted with a lower alkyl group].

2. The medical material according to claim 1, wherein said copolymer is a polyether-modified silicone.

3. The medical material according to claim 1 or 2 wherein said compound of the general formula (I) is (2R,4R)-4-methyl-1-((2S)-2-{[(3RS)-3-methyl-1,2,3,4-tetrahydroquinolin-8-yl]sulfonyl} amino-5-guanidinopentanoyl)piperidine-2-carboxylic acid.

4. The medical material according to any one of claims 1 to 3 comprising any selected from the group consisting of polyester, polyurethane, polystyrene, polymethyl methacrylate, polyvinyl chloride, polytetrafluoroethylene and polysulfone as a material.

5. The medical material according to any one of claims 1 to 4, which is a hollow fiber membrane.

6. A module for hemocatharsis filled with said medical material according to claim 5.

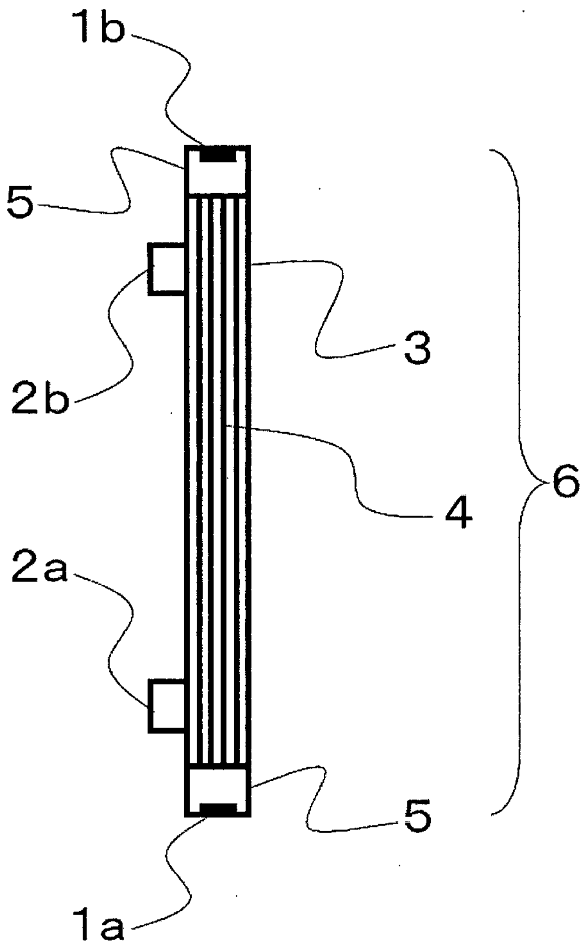


Fig.1

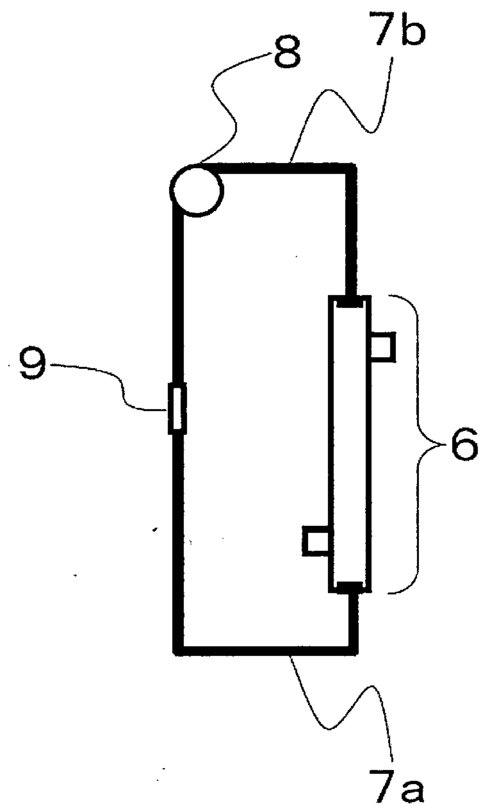


Fig.2

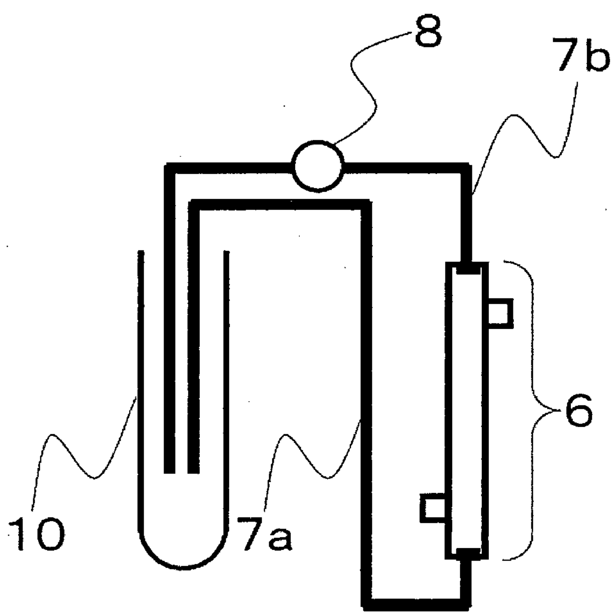


Fig.3

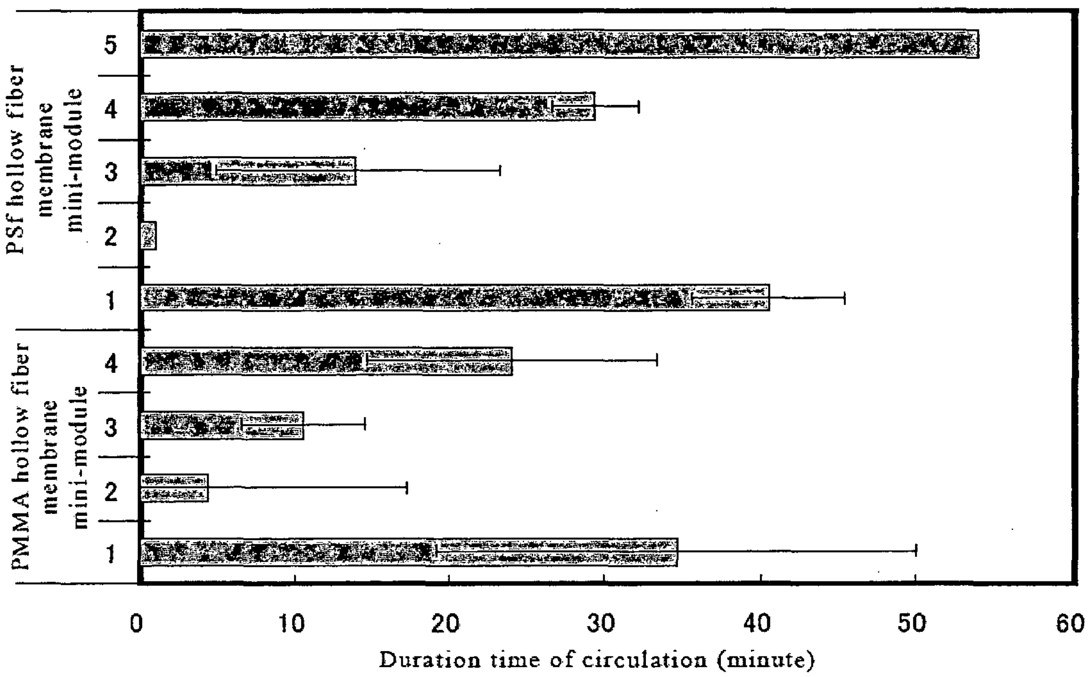
PSf hollow fiber membrane mini-module
PMMA hollow fiber membrane mini-module
5
4
3
2
1
4
3
2
1
0
10
20
30
40
50
60
Duration time of circulation (minute)

Fig.4

## INTERNATIONAL SEARCH REPORT

International application No.
PCT/JP2012/065946

**A. CLASSIFICATION OF SUBJECT MATTER**

*A61L33/00*(2006.01)i, *C08F216/06*(2006.01)i, *C08F218/08*(2006.01)i, *C08G65/333*(2006.01)i, *C08G65/334*(2006.01)i

According to International Patent Classification (IPC) or to both national classification and IPC

**B. FIELDS SEARCHED**

Minimum documentation searched (classification system followed by classification symbols)
A61L33/00, C08F216/06, C08F218/08, C08G65/333, C08G65/334

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY/MEDLINE/EMBASE/BIOSIS(STN), JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-225824 A (Toray Industries, Inc.), 08 October 2009 (08.10.2009), (Family: none) | 1-6 |
| Y | JP 2003-024452 A (Kawasumi Laboratories, Inc.), 28 January 2003 (28.01.2003), (Family: none) | 1-6 |
| Y | TSUKAGOSHI Tatsuya. et al, Protein adsorption on polymer-modified silica particle surface Colloids Surf B., 2007, Vol.54 No.1 p.101-107 | 1-6 |
| Y | Akitaka SENUMA, Hiroaki SHOJI, "Silicone aiming at new era. Technological development of modified silicone polymers", JETI., 1995, vol.43, no.12, pages 109 to 111 | 1-6 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| | |
|---|---|
| Date of the actual completion of the international search<br>08 August, 2012 (08.08.12) | Date of mailing of the international search report<br>21 August, 2012 (21.08.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/065946

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2011/078208 A1 (Toray Industries, Inc.), 30 June 2011 (30.06.2011), (Family: none) | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003507082 W
- JP 2001213984 A
- JP 2004525888 W
- JP 2006291193 A
- WO 08032758 A
- JP 2009225824 A
- JP 2010082067 A
- JP 2007181691 A
- JP 2007181692 A